# FASCICULE DE BREVET EUROPEEN

(11) **EP 3 942 705 B1**
(45) Date de publication et mention de la délivrance du brevet: **26.04.2023**
(21) Numéro de dépôt: 20710531.3
(22) Date de dépôt: 16.03.2020
(51) Int. Cl.: H04B 5/00, G06K 19/07, A01K 11/00, A61B 5/00, A01K 1/03, H01Q 1/22, G06K 19/077, A61B 5/11, G06K 7/10

(54) **PROCEDE, INSTALLATION ET ETIQUETTE POUR LE SUIVI DE L'ACTIVITE D'ANIMAUX EN CAPTIVITE**
VERFAHREN, EINRICHTUNG UND ETIKETT ZUR VERFOLGUNG DER AKTIVITÄT VON TIEREN IN GEFANGENSCHAFT
METHOD, FACILITY AND TAG FOR TRACKING THE ACTIVITY OF ANIMALS IN CAPTIVITY

(30) Priorité: 22.03.2019 FR 1903004
(43) Date de publication de la demande: 26.01.2022
(73) Titulaire: Centre national de la recherche scientifique, 75016 Paris (FR); Université Côte d'Azur, 06100 Nice Cedex 2 (FR)
(72) Inventeur: STARAJ, Robert, 06570 SAINT-PAUL DE VENCE (FR); CARLE, Georges, 06000 NICE (FR); PERRISOL, Philippe, 06650 OPIO (FR); LE THUC, Philippe, 06130 GRASSE (FR); DIALLO, Aliou, 06000 NICE (FR)
(74) Mandataire: IPAZ
(86) Numéro de dépôt international: PCT/EP2020/057103
(87) Numéro de publication internationale: WO 2020/193252

(56) Documents cités:
- US-A- 5 482 008
- US-A1- 2005 248 460
- US-A1- 2008 106 419
- US-A1- 2017 111 128
- US-A1- 2018 253 517

## Description

La présente invention a pour objet un procédé et une installation pour le suivi de l'activité d'animaux en hébergement confiné, ainsi qu'une étiquette destinée à équiper un animal en hébergement confiné.

La surveillance de l'activité d'animaux en hébergement confiné dans divers contextes, que ce soit en pré-opératoire, en post-opératoire, en clinique ou en laboratoire, requiert une observation quotidienne par une personne compétente.

L'utilisation de systèmes de surveillance automatisés et sans intervention humaine est dès lors préférable pour relever et analyser les signes vitaux des animaux, dans la mesure où ces systèmes ne perturbent pas l'animal, ne lui causent aucun préjudice, et n'augmentent pas son niveau d'anxiété.

Les techniques de surveillance permettent également d'obtenir des résultats plus précis, dans la mesure où ils s'affranchissent du "syndrome de la blouse blanche". Ainsi, il a été montré que la lecture des données vitales fournies par des implants invasifs, telles que la température, le taux de glucose et autres physiologies, par un intervenant humain à proximité de l'animal, était de fait perturbée.

Plus particulièrement, il a été établi que les animaux réagissent systématiquement négativement ou positivement à la proximité des humains et que l'acte même de tenter d'obtenir la lecture des données fournies par des implants invasifs, faussait les données.

Parallèlement à ce constat, le bien-être animal ainsi que la douleur et la détresse que les animaux utilisés à des fins scientifiques peuvent ressentir, sont depuis longtemps une préoccupation du grand public et de la communauté des chercheurs.

En effet il existe une Directive 2010/63/UE du Parlement européen et du Conseil du 22 septembre 2010 relative à la protection des animaux utilisés à des fins scientifiques, traduite en droit français dans le Décret n° 2013-118 du 1er février 2013 relatif à la protection des animaux utilisés à des fins scientifiques.

Ce sont ces préoccupations, combinées à l'utilisation accrue des animaux en recherche fondamentale et appliquée, qui ont motivé W. M. S. Russell et R. L. Burch à examiner comment les décisions devraient être prises quant à une telle utilisation des animaux.

Dans l'ouvrage The Principles of Humane Experimental Technique [les principes d'une technique expérimentale conforme à l'éthique], dont la première parution remonte à 1959, les auteurs Russell et Burch proposent de mettre en oeuvre le principe des Trois R, qui prône le remplacement, la réduction et le raffinement. Depuis une quarantaine d'années, ce principe des Trois R est largement reconnu comme un principe éthique établi dans le cadre de la science faisant appel à l'utilisation d'animaux.

Un certain nombre de travaux visant à développer des installations pour le suivi de l'activité d'animaux en hébergement confiné ont été conduits.

Ainsi, les documents US 2017/111128 A1 et US 2018/253517 A1 font état de procédés et d'installations pour le suivi de l'activité d'animaux en hébergement confiné dans une installation formée d'une cage, d'une pluralité d'antennes disposée sous la cage, un lecteur RFID auquel est reliée la pluralité d'antennes, des moyens informatiques aptes à piloter en émission et en réception le lecteur RFID, et une étiquette RFID destinée à équiper un animal en hébergement confiné dans la cage.

En plus, le document US 2008/106419 A1 divulgue des étiquettes qui peuvent être implantées à un petit animal.

Toutefois, dans le but d'améliorer la précision des données collectées, il s'est avéré nécessaire d'améliorer les étiquettes RFID destinées à équiper les animaux.

L'invention propose à cet effet une installation pour le suivi de l'activité d'animaux en hébergement confiné, l'installation comportant au moins une cage, une pluralité d'antennes disposée sous la au moins une cage, un lecteur auquel est reliée la pluralité d'antennes, des moyens informatiques aptes à piloter en émission et en réception le lecteur, et une étiquette destinée à équiper un animal en hébergement confiné dans la cage, l'étiquette comprenant une antenne et une puce encapsulées dans une gélule en matériau biocompatible, l'antenne présentant, en mode développé, une plaque centrale s'étendant longitudinalement et entourée d'une boucle ouverte dotée de deux languettes coudées.

Selon certaines caractéristiques optionnelles, la superficie de la cage est inférieure à 1 mètre, la cage mesurant entre 10 et 30 cm en largeur, et 25 à 75 cm en longueur.

Selon d'autres caractéristiques optionnelles, au moins quatre antennes sont réparties sous la cage, préférablement entre six et huit antennes.

De préférence, le lecteur est un lecteur RFID.

De préférence, l'étiquette est une étiquette RFID passive dotée d'une antenne apposée sur une puce associée à un identifiant.

De préférence, l'antenne et la puce sont encapsulées dans une gélule en matériau biocompatible de manière à ce que l'étiquette soit injectable par voie sous-cutanée.

L'invention a également pour objet une étiquette destinée à équiper un animal en hébergement confiné dans une installation conforme à un mode de réalisation de l'invention, l'étiquette comprenant une antenne et une puce encapsulées dans une gélule en matériau biocompatible, l'antenne présentant, en mode développé, une plaque centrale s'étendant longitudinalement et entourée d'une boucle ouverte dotée de deux languettes coudées.

Des caractéristiques optionnelles de l'invention, complémentaires ou de substitution sont énoncées ci-après.

Selon certaines caractéristiques optionnelles, la longueur de la boucle LLOOP est inférieure à 20 mm, préférentiellement sensiblement de l'ordre de 17,4 mm, la largeur de la boucle WLOOP est inférieure à 2 mm, préférentiellement sensiblement de l'ordre de 1,4 mm, la longueur de la plaque centrale LP est inférieure à 20 mm, préférentiellement sensiblement de l'ordre de 16,6 mm, la largeur de la plaque centrale WP est inférieure à 1 mm, préférentiellement sensiblement de l'ordre de 0,45 mm, la longueur des languettes coudées Lstub est inférieure à 1 mm, préférentiellement sensiblement de l'ordre de 0,9 mm.

De préférence, l'antenne est composée d'un matériau conducteur, préférentiellement du cuivre, tandis que la puce est composée d'un matériau composite, préférentiellement un verre renforcé en PTFE.

L'invention a également pour objet un procédé de suivi de l'activité d'animaux en hébergement confiné dans une installation comportant au moins une cage, une pluralité d'antennes disposée sous la au moins une cage, un lecteur RFID auquel est reliée la pluralité d'antennes, des moyens informatiques aptes à piloter en émission et en réception le lecteur RFID, et une étiquette RFID destinée à équiper un animal en hébergement confiné dans la cage, l'étiquette comprenant une antenne et une puce encapsulées dans une gélule en matériau biocompatible, l'antenne présentant, en mode développé, une plaque centrale s'étendant longitudinalement et entourée d'une boucle ouverte dotée de deux languettes coudées, procédé dans lequel
- l'animal est équipé de l'étiquette,
- le lecteur RFID génère au niveau de la pluralité d'antennes l'émission d'une onde électromagnétique,
- le lecteur RFID identifie laquelle (ou lesquelles) de la pluralité d'antennes a (ou ont) réceptionné la réponse émise par l'étiquette RFID,
de sorte que la position de l'animal est déterminée en fonction de la ou des antennes qui a ou qui ont réceptionné la réponse émise par l'étiquette RFID.

Selon certaines caractéristiques optionnelles, les moyens informatiques analysent sur une durée donnée quelles antennes ont réceptionné un signal émis par l'étiquette RFID et déterminent ainsi les positions successives de l'animal.

Selon d'autres caractéristiques optionnelles, les moyens informatiques déterminent la position de l'animal au moyen d'algorithmes basés sur les méthodes de triangulation ou bien au moyen d'algorithmes de positionnement par « fingerprinting ».

Selon d'autres caractéristiques optionnelles, la superficie de la cage est inférieure à 1 mètre, la cage mesurant entre 10 et 30 cm en largeur, et 25 à 75 cm en longueur. Selon d'autres caractéristiques optionnelles, au moins quatre antennes sont réparties sous la cage, préférablement entre six et huit antennes.

L'animal est équipé de l'étiquette par injection sous cutanée de ladite étiquette, ladite étiquette comportant une antenne et une puce encapsulées dans une gélule en matériau biocompatible.

Selon d'autres caractéristiques optionnelles, la longueur de la boucle LLOOP est inférieure à 20 mm, préférentiellement sensiblement de l'ordre de 17,4 mm, la largeur de la boucle WLOOP est inférieure à 2 mm, préférentiellement sensiblement de l'ordre de 1,4 mm, la longueur de la plaque centrale LP est inférieure à 20 mm, préférentiellement sensiblement de l'ordre de 16,6 mm, la largeur de la plaque centrale WP est inférieure à 1 mm, préférentiellement sensiblement de l'ordre de 0,45 mm, la longueur des languettes coudées Lstub est inférieure à 1 mm, préférentiellement sensiblement de l'ordre de 0,9 mm.

Selon d'autres caractéristiques optionnelles, l'antenne est composée d'un matériau conducteur, préférentiellement du cuivre, tandis que la puce est composée d'un matériau composite, préférentiellement un verre renforcé en PTFE.

Selon d'autres caractéristiques optionnelles, l'étiquette RFID émet des signaux véhiculant des informations sur l'état physiologique de l'animal.

D'autres avantages et particularités de l'invention apparaîtront à la lecture de la description détaillée de mises en oeuvre et de modes de réalisation nullement limitatifs, et des dessins annexés suivants :
[Fig.1] Cette figure est une vue schématique d'une installation pour le suivi de l'activité d'animaux en hébergement confiné, conforme à un mode de réalisation de l'invention.
[Fig.2] Cette figure est une vue de dessus d'un élément constitutif d'une étiquette destinée à équiper un animal en hébergement confiné, l'étiquette étant conforme à un mode de réalisation de l'invention.
[Fig.3] Cette figure est une vue en coupe transversale d'une étiquette destinée à équiper un animal en hébergement confiné, l'étiquette étant conforme à un mode de réalisation de l'invention.

A des fins de clarté et de concision, les références sur les figures correspondent aux mêmes éléments.

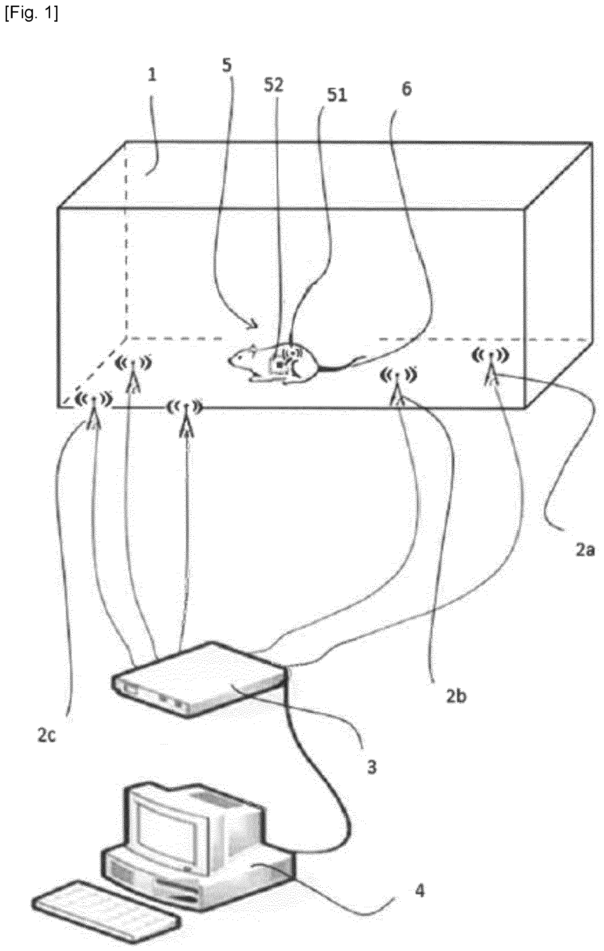

La Figure 1 décrit une installation pour le suivi de l'activité d'animaux en hébergement confiné selon un mode de réalisation de l'invention.

L'installation comporte une cage 1 de superficie inférieure à 1 mètre carré, c'est-à-dire une cage destinée à de petits animaux comme par exemple des rongeurs.

De préférence, la cage mesure entre 10 et 30 cm pour ce qui est de la largeur, et entre 25 et 75 cm pour ce qui est de la longueur.

Une pluralité d'antennes 2a, 2b, 2c est disposée sous la cage, et de préférence entre quatre et huit antennes.

La pluralité d'antennes 2a, 2b, 2c, est reliée à un lecteur 3 piloté en émission et en réception par des moyens informatiques 4.

Bien entendu, l'installation peut comporter une pluralité de cages, chacune des cages étant équipée d'un lot d'antennes disposées sous la cage.

L'ensemble des lots d'antennes peut être connecté à un seul et unique lecteur piloté en émission et en réception par des moyens informatiques centralisés.

L'installation comporte en outre une étiquette 5 destinée à équiper un animal en hébergement confiné dans la cage.

Cette étiquette est apte à réceptionner les signaux émis par les antennes disposées sous la cage lorsqu'elle se situe dans leur champ respectif.

Cette étiquette est également apte à réceptionner l'intensité des signaux (appelée niveau RSSI du signal) émis par les antennes disposées sous la cage.

Cette étiquette est également apte à émettre un signal en réponse qui est à son tour réceptionné par l'antenne émettrice et transmis au lecteur.

Le lecteur transmet ainsi aux moyens informatiques une succession d'informations qui indique quelles sont les antennes qui ont été sollicitées par la proximité de l'étiquette.

Il devient alors possible de déterminer approximativement les localisations successives de l'animal porteur de l'étiquette.

Le lecteur 3 est un lecteur RFID associé à l'étiquette 5 également RFID. De préférence, l'étiquette 5 est une étiquette RFID passive dotée d'une antenne 51 apposée sur une puce 52 associée à un identifiant.

L'antenne 51 et la puce 52 sont encapsulées dans une gélule en matériau biocompatible et de manière à ce que l'étiquette soit injectable par voie sous-cutanée.

En effet, pour implanter une étiquette dans un petit animal de laboratoire, il est préférable d'utiliser des injecteurs (ou seringues) adaptés car ils permettent de ne pas avoir à réaliser d'actes chirurgicaux, et d'être utilisés par des zootechniciens ne possédant pas d'habilitation pour la chirurgie.

On notera que la gélule, dite aussi capsule, peut contenir en plus d'une petite antenne d'autres composants tels que des capteurs, des puces électroniques voire accessoirement une caméra ou une batterie.

L'étiquette 5 pourra s'inspirer du format de la puce TAM-M^{®} de la société Intellibio. Cette étiquette a la forme d'une gélule de dimension 1,4 x 8 mm, en verre biocompatible et revêtue de parylène pour éviter la migration.

Elle offre une durée de vie bien supérieure à celle des petits animaux de laboratoire et présente de bonnes performances de lecture. Sa mémoire est programmable en écriture de 96 caractères alphanumériques.

Ainsi, l'étiquette peut intégrer un grand nombre d'informations sur l'état physiologique de l'animal.

L'étiquette 5, qui constitue un des objets de l'invention va maintenant être décrite plus en détail.

Il convient tout d'abord de rappeler que le suivi des petits animaux utilisés dans le cadre de tests de laboratoire et l'analyse de leur comportement à distance, à faible coût et en temps réel a fait l'objet de nombreux travaux de recherche.

Un axe majeur réside dans la problématique de l'implantation de capteurs sans fils miniatures qui nécessitent des composants de haute technicité dont le plus problématique est l'antenne.

En effet, la principale difficulté dans la conception d'antennes pour les dispositifs de communication bio-implantables est de fournir une structure rayonnante efficace et ce, malgré les contraintes de volume et le fort impact des tissus biologiques qui l'entourent.

Si de nombreuses études ont porté sur l'utilisation d'antennes implantées dans la bande MICS (Medical Implant Communications Service) (402-405 MHz), il faut noter qu'à ces fréquences, la taille des antennes peut être un inconvénient réel dans le cas de petits animaux, d'où une recherche de miniaturisation.

Une alternative consiste en l'utilisation de la technologie RFID (Radio-Frequency Identification) dans la bande UHF de 860 à 960 MHz.

En effet, elle présente deux avantages primordiaux : elle facilite l'implantation de l'étiquette et ne nécessite pas l'ajout d'une batterie pour alimenter le périphérique implanté.

Après l'analyse d'un bilan de liaison permettant de calculer les performances minimales de l'antenne implantée à concevoir pour une communication fiable et efficace, une étiquette RFID passive en 3D a été optimisée par différentes techniques pour atteindre des structures finales implantables ou injectables au dos d'une souris.

Cette étiquette RFID passive en 3D répond au cahier des charges suivant :
- Bande de fréquence RFID européenne : 865-868 MHz
- Dimensions maximales autorisées : 20 x 2 x 1 mm3
- Portée minimale : 5,8 cm
- Impédance de la puce utilisée : Impinj Monza R6 13-j126 Ohm
- Impédance de l'antenne proposée : 13+j126 Ohm

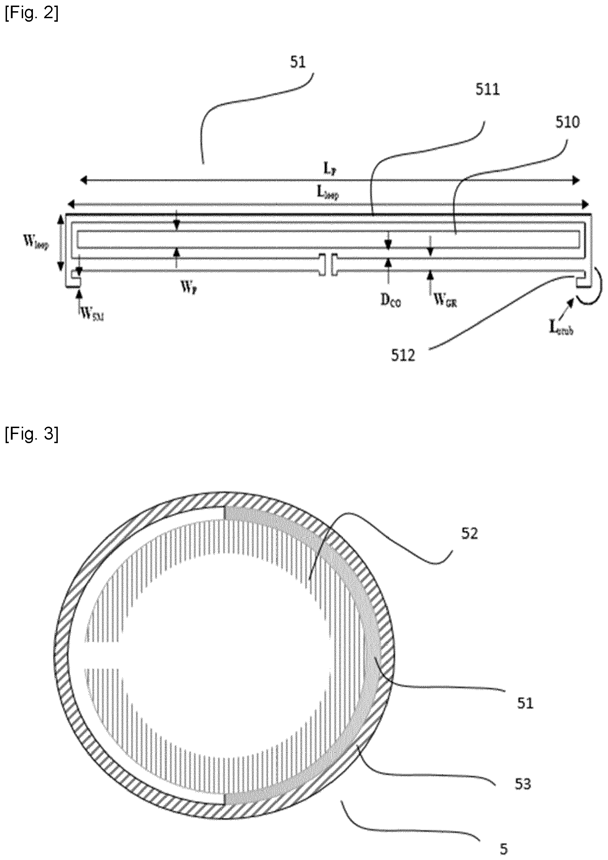

Tel que représenté sur la figure 3, l'étiquette comprend une antenne 51 et une puce 52 encapsulées dans une gélule 53.

La puce 52 est un substrat en matériau composite, en l'occurrence un verre renforcé en PTFE, soit par exemple du RG5880 Duroid, qui est doté d'une permittivité relative de l'ordre de 2,2, d'une tangente de perte de l'ordre de 0,0009 et d'une épaisseur de l'ordre de 0,127 mm.

La capsule (ou gélule) devant être biocompatible, elle peut être réalisée à l'aide d'un tube de verre borosilicaté précontraint, qui présente avantageusement une grande résistance aux chocs et aux rayures.

Ce matériau a une permittivité relative de l'ordre de 4,6, une tangente de pertes de l'ordre de 0,0037, une épaisseur de l'ordre de 0,1 mm et un diamètre de l'ordre de 1,6 mm.

La couche de verre borosilicaté a pour fonction d'empêcher la possibilité de rejet de l'implant par le corps animal, et également de faciliter la transition de l'onde rayonnante entre l'antenne implantée et les tissus animaux.

Tel que représenté sur la figure 2, l'antenne 51 est fabriquée à partir d'une plaque de cuivre.

L'antenne 51 présente, en mode développé, une plaque centrale 510 entourée d'une boucle ouverte 511 dotée de deux languettes coudées 512.

La longueur de la boucle 511 LLOOP est inférieure à 20 mm, préférentiellement sensiblement de l'ordre de 17,4 mm.

La largeur de la boucle WLOOP est inférieure à 2 mm, préférentiellement sensiblement de l'ordre de 1,4 mm.

La longueur de la plaque centrale 510 LP est inférieure à 20 mm, préférentiellement sensiblement de l'ordre de 16,6 mm.

La largeur de la plaque centrale WP est inférieure à 1 mm, préférentiellement sensiblement de l'ordre de 0,45 mm.

La longueur des languettes coudées Lstub est inférieure à 1 mm, préférentiellement sensiblement de l'ordre de 0,9 mm.

Cette géométrie, qui correspond à un volume total très faible pour l'antenne (puisque de l'ordre de 36,2 mm³) permet d'obtenir une impédance de 13,4 + j126,3 Ohms avec un gain total de -23,4 dBi et une efficacité de 0,2 %.

Ces valeurs sont très acceptables par rapport aux antennes classiques qui sont plus volumineuses et qui fonctionnent à ces fréquences.

Le procédé de suivi de l'activité d'animaux en hébergement confiné dans une installation telle que décrite précédemment, va maintenant être décrit, étant entendu qu'on dispose d'au moins un petit animal confiné dans une cage 1 de superficie inférieure à 1 mètre carré, une pluralité d'antennes 2a, 2b, 2c étant disposée sous la cage, et reliées à un lecteur RFID 3, des moyens informatiques 4 étant aptes à piloter en émission et en réception le lecteur RFID.

Selon une étape préalable, il est nécessaire d'équiper l'animal avec l'étiquette 5.

Puis, le lecteur RFID 3 génère au niveau de la pluralité d'antennes 2a, 2b, 2c l'émission d'une onde électromagnétique.

Puis, le lecteur RFID 3 identifie laquelle ou lesquelles de la pluralité d'antennes a ou ont réceptionné la réponse émise par l'étiquette RFID, de sorte que la position de l'animal est déterminée en fonction de la ou des antennes qui a ou qui ont réceptionné la réponse émise par l'étiquette RFID.

Les moyens informatiques 4 sont dès lors en mesure d'analyser sur une durée donnée quelles antennes ont réceptionné un signal émis par l'étiquette RFID et déterminent ainsi les positions successives de l'animal.

Les moyens informatiques 4 peuvent alors déterminer la position de l'animal au moyen d'algorithmes basés sur les méthodes de triangulation ou bien au moyen d'algorithmes de positionnement par « fingerprinting », ou bien au moyen d'algorithmes de positionnement par « WLAN Indoor Positionning Algorithm ».

Ces algorithmes utilisent le RSSI (Received Signal Strength Indication) qui est une valeur qui quantifie le niveau d'énergie des ondes électromagnétiques (l'intensité du signal mesuré) reçu par l'antenne de réception. Plus la source est située à proximité, plus le signal radio est fort et plus le niveau du RSSI est élevé.

Le RSSI est estimé différemment en fonction des technologies de télécommunication utilisées. Par exemple, pour le réseau mobile 4G, le RSSI est mesuré seulement sur les porteuses attribuées, tandis que pour le Wi-Fi ou le réseau 3G, il est nécessaire de le mesurer seulement sur la bande de fréquence.

En ce qui concerne la technologie RFID, la valeur du RSSI est mesurée dans toute sa bande de fréquence attribuée.

Les valeurs peuvent alors être obtenues par un logiciel sur un ordinateur local.

Il est possible de conclure que le système fonctionne correctement lorsque la valeur reçue à partir de l'étiquette est supérieure à la sensibilité du lecteur.

Concernant maintenant les algorithmes, ceux du type «fingerprinting», et ceux du type « WLAN Indoor Positionning Algorithm » sont particulièrement adaptés dans le cas des problématiques de géolocalisation en intérieur sur des espaces confinés.

L'approche par « empreinte radio » ("fingerprinting signal") comprend deux étapes: l'étalonnage et la localisation.

La phase d'étalonnage comprend l'acquisition de caractéristiques (généralement l'intensité appelée RSSI) des signaux issus des émetteurs stationnaires (balises) en des points prédéfinis, qui sont utilisés pour construire une base de données qui correspond aux valeurs collectées (empreintes digitales) avec les emplacements correspondants.

Au cours de la phase de localisation, le dispositif mobile acquiert une empreinte digitale du signal et le système de positionnement utilise les données d'étalonnage, couplées à des algorithmes appropriés pour déterminer la meilleure correspondance pour l'emplacement auquel l'empreinte digitale appartient le plus probablement.

L'approche avec l'algorithme de positionnement WLAN (Wireless Local Area Network) utilise un système de communication très répandu (plus connu sous le nom de « réseau Wi-Fi.

Cet algorithme se compose de deux phases : "off-line measurement" et "on-line measurement" qui intègrent également différents algorithmes de positionnements du type NN (Nearest-Neighbor) ou KNN (K Nearest-Neighbour).

Ainsi, l'installation et le procédé selon l'invention rendent possible l'enregistrement avec précision des déplacements d'un animal de laboratoire en hébergement confiné.

Les données recueillies par l'installation peuvent également être mises au service des technologies d'intelligence artificielle.

Il devient ainsi possible d'analyser le comportement et les mouvements des petits animaux de laboratoire en utilisant l'apprentissage en profondeur (deep learning).

Il s'agit d'une technologie d'intelligence artificielle visant à anticiper des comportements particuliers sur la base d'un certain nombre d'informations.

Ces données permettent de mettre en évidence par exemple les stéréotypies de ces animaux, les stéréotypies étant classiquement définies comme des comportements répétitifs, invariants et qui n'ont aucun but ou fonction apparents.

A partir de ces données, il est par exemple possible de déterminer si l'activité de l'animal est une manifestation réactionnelle ou pathologique, comportementale ou organique.

A noter, les différentes caractéristiques, formes, variantes et modes de réalisation de l'invention peuvent être associés les uns avec les autres, selon diverses combinaisons dans la mesure où ils ne sont pas incompatibles avec la portée des revendications ci-jointes.

## Revendications

1. Etiquette (5) destinée à équiper un animal en hébergement confiné, dans une installation pour le suivi de l'activité d'animaux en hébergement confiné, l'installation comportant au moins une cage (1), une pluralité d'antennes (2a, 2b, 2c) disposée sous la au moins une cage, un lecteur (3) auquel est reliée la pluralité d'antennes (2a, 2b, 2c), et des moyens informatiques (4) aptes à piloter en émission et en réception le lecteur, l'étiquette (5) comprenant une antenne (51) et une puce (52) encapsulées dans une gélule en matériau biocompatible (53),
**caractérisée en ce que**
l'antenne (51) présente, en mode développé, une plaque centrale (510) s'étendant longitudinalement et entourée d'une boucle ouverte (511) dotée de deux languettes coudées (512).

2. Etiquette (5) destinée à équiper un animal en hébergement confiné, selon la revendication 1, **caractérisée en ce que** la longueur de la boucle (511), LLOOP, est inférieure à 20 mm, préférentiellement sensiblement de l'ordre de 17,4 mm, la largeur de la boucle, WLOOP, est inférieure à 2 mm, préférentiellement sensiblement de l'ordre de 1,4 mm, la longueur de la plaque centrale (510), LP, est inférieure à 20 mm, préférentiellement sensiblement de l'ordre de 16,6 mm, la largeur de la plaque centrale, WP, est inférieure à 1 mm, préférentiellement sensiblement de l'ordre de 0,45 mm, la longueur des languettes coudées, Lstub, est inférieure à 1 mm, préférentiellement sensiblement de l'ordre de 0,9 mm.

3. Etiquette (5) destinée à équiper un animal en hébergement confiné, selon les revendications 1 ou 2, **caractérisée en ce que** l'antenne (51) est composée d'un matériau conducteur, préférentiellement du cuivre, tandis que la puce (52) est composée d'un matériau composite, préférentiellement un verre renforcé en PTFE.

4. Installation pour le suivi de l'activité d'animaux en hébergement confiné, l'installation comportant au moins une cage (1), une pluralité d'antennes (2a, 2b, 2c) disposée sous la au moins une cage, un lecteur (3) auquel est reliée la pluralité d'antennes (2a, 2b, 2c), des moyens informatiques (4) aptes à piloter en émission et en réception le lecteur, et une étiquette (5) RFID destinée à équiper un animal en hébergement confiné dans la cage, l'étiquette (5) étant conforme à l'une quelconque des revendications 1 à 3.

5. Installation pour le suivi de l'activité d'animaux en hébergement confiné selon la revendication 4, **caractérisée en ce que** la superficie de la cage est inférieure à 1 mètre, la cage mesurant entre 10 et 30 cm en largeur, et 25 à 75 cm en longueur.

6. Installation pour le suivi de l'activité d'animaux en hébergement confiné selon la revendication 4 ou 5, **caractérisée en ce qu'**au moins quatre antennes (2a, 2b, 2c) sont réparties sous la cage.

7. Installation pour le suivi de l'activité d'animaux en hébergement confiné selon la revendication 6, **caractérisée en ce qu'**entre six et huit antennes sont réparties sous la cage.

8. Installation pour le suivi de l'activité d'animaux en hébergement confiné selon l'une quelconque des revendications 4 à 7, **caractérisée en ce que** le lecteur (3) est un lecteur RFID associé à une étiquette RFID.

9. Procédé de suivi de l'activité d'animaux en hébergement confiné dans une installation comportant au moins une cage (1), une pluralité d'antennes (2a, 2b, 2c) disposée sous la au moins une cage, un lecteur RFID (3) auquel est reliée la pluralité d'antennes (2a, 2b, 2c), des moyens informatiques (4) aptes à piloter en émission et en réception le lecteur RFID, et une étiquette RFID (5) destinée à équiper un animal en hébergement confiné dans la cage, l'étiquette (5) étant conforme à la revendication 1, procédé dans lequel :
- l'animal est équipé de l'étiquette (5) par injection sous-cutanée,
- le lecteur RFID (3) génère au niveau de la pluralité d'antennes (2a, 2b, 2c) l'émission d'une onde électromagnétique ,
- le lecteur RFID (3) identifie laquelle (ou lesquelles) de la pluralité d'antennes a (ou ont) réceptionné la réponse émise par l'étiquette RFID, de sorte que la position de l'animal est déterminée en fonction de la ou des antennes qui a ou qui ont réceptionné la réponse émise par l'étiquette RFID.

10. Procédé de suivi de l'activité d'animaux en hébergement confiné selon la revendication 9, **caractérisé en ce que** les moyens informatiques (4) analysent sur une durée donnée quelles antennes ont réceptionné un signal émis par l'étiquette RFID et déterminent ainsi les positions successives de l'animal.

11. Procédé de suivi de l'activité d'animaux en hébergement confiné selon la revendication 10, **caractérisé en ce que** les moyens informatiques (4) déterminent la position de l'animal au moyen d'algorithmes basés sur les méthodes de triangulation ou bien au moyen d'algorithmes de positionnement par « fingerprinting ».

12. Procédé de suivi de l'activité d'animaux en hébergement confiné selon l'une quelconque des revendications 9 à 11, **caractérisé en ce que** la superficie de la cage est inférieure à 1 mètre, la cage mesurant entre 10 et 30 cm en largeur, et 25 à 75 cm en longueur.

13. Procédé de suivi de l'activité d'animaux en hébergement confiné selon l'une quelconque des revendications 9 à 12, **caractérisé en ce qu'**au moins quatre antennes (2a, 2b, 2c) sont réparties sous la cage.

14. Procédé de suivi de l'activité d'animaux en hébergement confiné selon la revendication 13, **caractérisé en ce qu'**entre six et huit antennes sont réparties sous la cage.

15. Procédé de suivi de l'activité d'animaux en hébergement confiné selon la revendication 9, **caractérisée en ce que** la longueur de la boucle (511), LLOOP, est inférieure à 20 mm, préférentiellement sensiblement de l'ordre de 17,4 mm, la largeur de la boucle, WLOOP, est inférieure à 2 mm, préférentiellement sensiblement de l'ordre de 1,4 mm, la longueur de la plaque centrale (510), LP, est inférieure à 20 mm, préférentiellement sensiblement de l'ordre de 16,6 mm, la largeur de la plaque centrale, WP, est inférieure à 1 mm, préférentiellement sensiblement de l'ordre de 0,45 mm, la longueur des languettes coudées, Lstub, est inférieure à 1 mm, préférentiellement sensiblement de l'ordre de 0,9 mm.

16. Procédé de suivi de l'activité d'animaux en hébergement confiné selon la revendication 9, **caractérisée en ce que** l'antenne (51) est composée d'un matériau conducteur, préférentiellement du cuivre, tandis que la puce (52) est composée d'un matériau composite, préférentiellement un verre renforcé en PTFE.

17. Procédé de suivi de l'activité d'animaux en hébergement confiné selon l'une quelconque des revendications 9 à 16, **caractérisée en ce que** l'étiquette RFID émet des signaux véhiculant des informations sur l'état physiologique de l'animal.

## Patentansprüche

1. Etikett (5) zur Ausstattung eines Tieres in geschlossener Unterbringung in einer Einrichtung zur Nachverfolgung der Aktivität von Tieren in geschlossener Unterbringung, wobei die Einrichtung zumindest einen Käfig (1), mehrere Antennen (2a, 2b, 2c), die unter dem zumindest einen Käfig angeordnet sind, ein Lesegerät (3), mit dem die mehreren Antennen (2a, 2b, 2c) verbunden sind, und Computermittel (4) enthält, die geeignet sind, das Lesegerät zum Senden und Empfangen anzusteuern, wobei das Etikett (5) eine Antenne (51) und einen Chip (52) enthält, die in einer Kapsel aus biokompatiblem Material (53) eingekapselt sind,
**dadurch gekennzeichnet, dass**
die Antenne (51) im ausgefahrenen Modus eine Mittelplatte (510) aufweist, die sich in Längsrichtung erstreckt und von einer offenen Schlaufe (511) umgeben ist, die mit zwei abgewinkelten Lappen (512) versehen ist.

2. Etikett (5) zur Ausstattung eines Tieres in geschlossener Unterbringung nach Anspruch 1,
**dadurch gekennzeichnet, dass** die Länge der Schlaufe (511), LLOOP, unter 20 mm, vorzugsweise im Wesentlichen in der Größenordnung von 17,4 mm, liegt, die Breite der Schlaufe, WLOOP, unter 2 mm, vorzugsweise im Wesentlichen in der Größenordnung von 1,4 mm, liegt, die Länge der Mittelplatte (510), LP, unter 20 mm, vorzugsweise im Wesentlichen in der Größenordnung von 16,6 mm, liegt, die Breite der Mittelplatte, WP, unter 1 mm, vorzugsweise im Wesentlichen in der Größenordnung von 0,45 mm, liegt, und die Länge der abgewinkelten Lappen, Lstub, unter 1 mm, vorzugsweise im Wesentlichen in der Größenordnung von 0,9 mm, liegt.

3. Etikett (5) zur Ausstattung eines Tieres in geschlossener Unterbringung nach Anspruch 1 oder 2,
**dadurch gekennzeichnet, dass** die Antenne (51) aus einem leitenden Material, vorzugsweise Kupfer, besteht, während der Chip (52) aus einem Verbundmaterial, vorzugsweise PTFE-verstärktem Glas, besteht.

4. Einrichtung zur Nachverfolgung der Aktivität von Tieren in geschlossener Unterbringung, wobei die Einrichtung zumindest einen Käfig (1), mehrere Antennen (2a, 2b, 2c), die unter dem zumindest einen Käfig angeordnet sind, ein Lesegerät (3), mit dem die mehreren Antennen (2a, 2b, 2c) verbunden ist, Computermittel (4), die geeignet sind, das Lesegerät zum Senden und Empfangen anzusteuern, und ein RFID-Etikett (5) enthält, das dazu bestimmt ist, ein Tier in geschlossener Unterbringung in dem Käfig auszustatten, wobei das Etikett (5) einem der Ansprüche 1 bis 3 entspricht.

5. Einrichtung zur Nachverfolgung der Aktivität von Tieren in geschlossener Unterbringung nach Anspruch 4,
**dadurch gekennzeichnet, dass** die Fläche des Käfigs weniger als 1 Meter beträgt, wobei der Käfig zwischen 10 und 30 cm in der Breite und 25 bis 75 cm in der Länge misst.

6. Einrichtung zur Nachverfolgung der Aktivität von Tieren in geschlossener Unterbringung nach Anspruch 4 oder 5,
**dadurch gekennzeichnet, dass** zumindest vier Antennen (2a, 2b, 2c) unter dem Käfig verteilt sind.

7. Einrichtung zur Nachverfolgung der Aktivität von Tieren in geschlossener Unterbringung nach Anspruch 6,
**dadurch gekennzeichnet, dass** zwischen sechs und acht Antennen unter dem Käfig verteilt sind.

8. Einrichtung zur Nachverfolgung der Aktivität von Tieren in geschlossener Unterbringung nach einem der Ansprüche 4 bis 7,
**dadurch gekennzeichnet, dass** das Lesegerät (3) ein RFID-Lesegerät ist, dem ein RFID-Etikett zugeordnet ist.

9. Verfahren zur Nachverfolgung der Aktivität von Tieren in geschlossener Unterbringung in einer Einrichtung, die zumindest einen Käfig (1), mehrere Antennen (2a, 2b, 2c), die unter dem zumindest einen Käfig angeordnet sind, ein RFID-Lesegerät (3), mit dem die mehreren Antennen (2a, 2b, 2c) verbunden sind, Computermittel (4), die geeignet sind, das RFID-Lesegerät zum Senden und Empfangen anzusteuern, und ein RFID-Etikett (5) zum Ausstatten eines Tier in geschlossener Unterbringung in dem Käfig enthält, wobei das Etikett (5) dem Anspruch 1 entspricht, bei welchem Verfahren:
- das Tier durch subkutane Injektion mit dem Etikett (5) ausgestattet wird,
- das RFID-Lesegerät (3) im Bereich der mehreren Antennen (2a, 2b, 2c) die Aussendung einer elektromagnetischen Welle erzeugt,
- das RFID-Lesegerät (3) erkennt, welche der mehreren Antennen die von dem RFID-Etikett ausgesendete Antwort empfangen hat (haben), so dass die Position des Tieres in Abhängigkeit von der Antenne oder den Antennen bestimmt wird, die die von dem RFID-Etikett ausgesendete Antwort empfangen hat bzw. haben.

10. Verfahren zur Nachverfolgung der Aktivität von Tieren in geschlossener Unterbringung nach Anspruch 9,
**dadurch gekennzeichnet, dass** die Computermittel (4) über einen gegebenen Zeitraum analysieren, welche Antennen ein von dem RFID-Etikett ausgesendetes Signal empfangen haben, und so die aufeinanderfolgenden Positionen des Tieres bestimmen.

11. Verfahren zur Nachverfolgung der Aktivität von Tieren in geschlossener Unterbringung nach Anspruch 10,
**dadurch gekennzeichnet, dass** die Computermittel (4) die Position des Tieres mithilfe von Algorithmen bestimmen, die auf Triangulationsmethoden basieren, oder auch mithilfe von Algorithmen zur Positionierung durch "Fingerprinting".

12. Verfahren zur Nachverfolgung der Aktivität von Tieren in geschlossener Unterbringung nach einem der Ansprüche 9 bis 11,
**dadurch gekennzeichnet, dass** die Fläche des Käfigs weniger als 1 Meter beträgt, wobei der Käfig zwischen 10 und 30 cm in der Breite und 25 bis 75 cm in der Länge misst.

13. Verfahren zum Nachverfolgung der Aktivität von Tieren in geschlossener Unterbringung nach einem der Ansprüche 9 bis 12,
**dadurch gekennzeichnet, dass** zumindest vier Antennen (2a, 2b, 2c) unter dem Käfig verteilt sind.

14. Verfahren zur Nachverfolgung der Aktivität von Tieren in geschlossener Unterbringung nach Anspruch 13,
**dadurch gekennzeichnet, dass** zwischen sechs und acht Antennen unter dem Käfig verteilt sind.

15. Verfahren zur Nachverfolgung der Aktivität von Tieren in geschlossener Unterbringung nach Anspruch 9,
**dadurch gekennzeichnet, dass** die Länge der Schlaufe (511), LLOOP, unter 20 mm, vorzugsweise im Wesentlichen in der Größenordnung von 17,4 mm, liegt, die Breite der Schlaufe, WLOOP, unter 2 mm, vorzugsweise im Wesentlichen in der Größenordnung von 1,4 mm, liegt, die Länge der Mittelplatte (510), LP, unter 20 mm, vorzugsweise im Wesentlichen in der Größenordnung von 16,6 mm, liegt, die Breite der Mittelplatte, WP, unter 1 mm, vorzugsweise im Wesentlichen in der Größenordnung von 0,45 mm, liegt, und die Länge der abgewinkelten Lappen, Lstub, unter 1 mm, vorzugsweise im Wesentlichen in der Größenordnung von 0,9 mm, liegt.

16. Verfahren zur Nachverfolgung der Aktivität von Tieren in geschlossener Unterbringung nach Anspruch 9,
**dadurch gekennzeichnet, dass** die Antenne (51) aus einem leitenden Material, vorzugsweise Kupfer, besteht, während der Chip (52) aus einem Verbundmaterial, vorzugsweise PTFE-verstärktem Glas, besteht.

17. Verfahren zur Nachverfolgung der Aktivität von Tieren in geschlossener Unterbringung nach einem der Ansprüche 9 bis 16,
**dadurch gekennzeichnet, dass** das RFID-Etikett Signale aussendet, die Informationen über den physiologischen Zustand des Tieres übermitteln.

## Claims

1. Tag (5) intended to equip an animal housed in confined conditions, in an facility for tracking the activity of animals in confined conditions, the facility comprising at least one cage (1), a plurality of antennas (2a, 2b, 2c) arranged under the at least one cage, a reader (3) to which the plurality of antennas (2a, 2b, 2c) is connected, and computer means (4) capable of controlling the transmission and reception of the reader, the tag (5) comprising an antenna (51) and a chip (52) encapsulated in a gelatinous capsule, and the reader (3) being connected to the antenna (51), and computer means (4) capable of controlling the transmission and reception of the reader, the tag (5) comprising an antenna (51) and a chip (52) encapsulated in a capsule made of biocompatible material (53), **characterized in that** the antenna (51) has, in the developed mode, a central plate (510) extending longitudinally and surrounded by an open loop (511) provided with two angled tabs (512).

2. Tag (5) intended to equip an animal housed in confined conditions, according to claim 1, **characterized in that** the length of the loop (511) LLOOP is less than 20 mm, preferably substantially of the order of 17.4 mm, the width of the loop WLOOP is less than 2 mm, preferably substantially of the order of 1.4 mm the length of the central plate (510) LP is less than 20 mm, preferably substantially of the order of 16.6 mm, the width of the central plate WP is less than 1 mm, preferably substantially of the order of 0.45 mm, the length of the angled tabs Lstub is less than 1 mm, preferably substantially of the order of 0.9 mm.

3. Tag (5) for equipping an animal housed in a confined accommodation, according to claims 1 or 2, **characterized in that** the antenna (51) is composed of a conductive material, preferably copper, while the chip (52) is composed of a composite material, preferably a PTFE-reinforced glass.

4. Facility for tracking the activity of animals housed in confined conditions, the facility comprising at least one cage (1), a plurality of antennas (2a, 2b, 2c) arranged under the at least one cage, a reader (3) to which the plurality of antennas (2a, 2b, 2c), computer means (4) capable of controlling the transmission and reception of the reader, and an RFID tag (5) intended to equip an animal confined in the cage, the tag (5) being in accordance with any one of claims 1 to 3.

5. Facility for tracking the activity of animals housed in confined conditions according to claim 4, **characterized in that** the surface area of the cage is less than 1 meter, the cage measuring between 10 and 30 cm in width, and 25 to 75 cm in length.

6. Facility for tracking the activity of animals housed in confined conditions according to claim 4 or 5, **characterized in that** at least four antennas (2a, 2b, 2c) are distributed under the cage.

7. Facility for tracking the activity of animals housed in confined conditions according to claim 6, **characterized in that** between six and eight antennas are distributed under the cage.

8. Facility for tracking the activity of animals housed in confined conditions according to any one of claims 4 to 7, **characterized in that** the reader (3) is an RFID reader associated with an RFID tag.

9. Method for monitoring the activity of animals confined in a facility comprising at least one cage (1), a plurality of antennas (2a, 2b, 2c) arranged under the at least one cage, an RFID reader (3) to which the plurality of antennas (2a, 2b, 2c), computer means (4) capable of controlling the RFID reader in transmission and reception, and an RFID tag (5) intended to equip an animal in confined conditions in the cage, the tag (5) being in accordance with claim 1, in which method :
- the animal is equipped with the tag (5) by subcutaneous injection,
- the RFID reader (3) generates at the plurality of antennas (2a, 2b, 2c) the emission of an electromagnetic wave,
- the RFID reader (3) identifies which of the plurality of antennas has (or have) received the response emitted by the RFID tag,
so that the position of the animal is determined based on which of the antenna(s) has (have) received the response emitted by the RFID tag.

10. Method of monitoring the activity of animals in confined conditions according to claim 9, **characterized in that** the computer means (4) analyze over a given period of time which antennas have received a signal emitted by the RFID tag and thus determine the successive positions of the animal.

11. Method for monitoring the activity of animals in confined conditions according to claim 10, **characterized in that** the computer means (4) determine the position of the animal by means of algorithms based on triangulation methods or by means of fingerprinting positioning algorithms.

12. Method for monitoring the activity of animals in confined conditions according to any of claims 9 to 11, **characterized in that** the surface area of the cage is less than 1 meter, the cage measuring between 10 and 30 cm in width, and 25 to 75 cm in length.

13. Method of monitoring the activity of animals in confined conditions according to any one of claims 9 to 12, **characterized in that** at least four antennas (2a, 2b, 2c) are distributed under the cage.

14. Method of monitoring the activity of animals in confined conditions according to claim 13, **characterized in that** between six and eight antennas are distributed under the cage.

15. Method for monitoring the activity of animals in confined conditions according to claim 9, **characterized in that** the length of the loop (511) LLOOP is less than 20 mm, preferably substantially of the order of 17.4 mm, the width of the loop WLOOP is less than 2 mm, preferably substantially of the order of 1.4 mm, the length of the central plate (510) LP is less than 20 mm, preferably substantially of the order of 16.6 mm, the width of the central plate WP is less than 1 mm, preferably substantially of the order of 0.45 mm, the length of the angled tabs Lstub is less than 1 mm, preferably substantially of the order of 0.9 mm.

16. Method for monitoring the activity of animals in confined conditions according to claim 9, **characterized in that** the antenna (51) is composed of a conductive material, preferably copper, while the chip (52) is composed of a composite material, preferably a PTFE-reinforced glass.

17. Method for monitoring the activity of animals in confined conditions according to any one of claims 9 to 16, **characterized in that** the RFID tag emits signals conveying information on the physiological state of the animal.
